# EUROPEAN PATENT SPECIFICATION

(11) **EP 2 982 983 B1**
(45) Date of publication and mention of the grant of the patent: **01.03.2017**
(21) Application number: 15179738.8
(22) Date of filing: 04.08.2015
(51) Int. Cl.: G01N 33/569

(54) **METHOD FOR DRUG SUSCEPTIBILITY TESTING OF MYCOBACTERIUM TUBERCULOSIS BY ELECTRON PARAMAGNETIC RESONANCE**
VERFAHREN ZUR ARZNEIMITTELANFÄLLIGKEITSPRÜFUNG VON MYCOBACTERIUM TUBERCULOSIS DURCH PARAMAGNETISCHE ELEKTRONENRESONANZ
PROCÉDÉ DE TEST DE SENSIBILITÉ AUX MÉDICAMENTS DE MYCOBACTERIUM TUBERCULOSIS PAR RÉSONANCE PARAMAGNÉTIQUE ÉLECTRONIQUE

(30) Priority: 04.08.2014 IT RM20140454
(43) Date of publication of application: 10.02.2016
(73) Proprietor: ISTITUTO SUPERIORE DI SANITA', 00161 Roma (IT)
(72) Inventor: GIANNONI, Federico, 00199 Rome (IT); FATTORINI, Lanfranco, 05018 Orvieto (TR) (IT); PIETRAFORTE, Donatella, 00139 Rome (IT); PICCARO, Giovanni, Roccagorga (LT) (IT)
(74) Representative: Banchetti, Marina

(56) References cited:
- WO-A1-2005/078131
- P. BEMER ET AL: "Multicenter Evaluation of the MB/BACT System for Susceptibility Testing of Mycobacterium tuberculosis", JOURNAL OF CLINICAL MICROBIOLOGY, vol. 42, no. 3, 1 March 2004 (2004-03-01), pages 1030-1034, XP055180828, ISSN: 0095-1137, DOI: 10.1128/JCM.42.3.1030-1034.2004
- GUNEY ET AL: "P1642 Evaluation of the BACT/ALERT 3D system for testing susceptibility of Mycobacterium tuberculosis tofirst-line antituberculosis agents: comparison with BacTec MGIT 960 and Lowenstein-Jensen proportion method", INTERNATIONAL JOURNAL OF ANTIMICROBIAL AGENTS, ELSEVIER SCIENCE, AMSTERDAM, NL, vol. 29, 1 March 2007 (2007-03-01), page S463, XP022038831, ISSN: 0924-8579, DOI: 10.1016/S0924-8579(07)71481-4
- W. B. DAVIS ET AL: "Differentiation of Catalases in Mycobacterium phlei on the Basis of Susceptibility to Isoniazid: Association with Peroxidase and Acquired Resistance to Isoniazid", ANTIMICROBIAL AGENTS AND CHEMOTHERAPY, vol. 12, no. 4, 1 October 1977 (1977-10-01), pages 529-533, XP055180878, ISSN: 0066-4804, DOI: 10.1128/AAC.12.4.529

## Description

### Field of the invention

The present invention concerns a method for drug susceptibility testing of *Mycobacterium tuberculosis* by electron paramagnetic resonance (EPR). More specifically, the invention relates to a diagnostic method for the performance of antibiograms which allows to detect the activity of a tested antibacterial drug against a M. *tuberculosis* strain through the measurement of free radicals produced by such activity, by acquiring the EPR spectra of specific spin-adducts obtained by adding specific spin trapping reagents to the bacterial lysate after incubation of the bacteria with the drug under test.

### Background of the invention

Tuberculosis (TB) is a contagious airborne disease caused by *Mycobacterium tuberculosis.* An estimate of 8.7 millions of new tuberculosis cases and 1.4 million deaths occurs per year (**1**). The current therapy of TB caused by drug-susceptible (DS) *M. tuberculosis* strains includes administration of the first-line drugs rifampicin (also known as rifampin), isoniazid, ethambutol and pyrazinamide as follows: two months of rifampicin + isoniazid + ethambutol + pyrazinamide, followed by four months of rifampicin + isoniazid. Second-line drugs, which are used for the treatment of TB caused by multi-drug resistant (MDR) *M. tuberculosis* strains (*i.e.* strains resistant to at least rifampicin and isoniazid, the two major anti-TB drugs), include fluoroquinolones (ofloxacin and moxifloxacin), injectable drugs (amikacin, kanamycin, capreomycin) and other drugs, such as linezolid, clofazimine, and meropenem.

In the last decade, increasing isolation of MDR strains and extensively drug-resistant (XDR) *M. tuberculosis* strains (*i*.*e*., MDR also resistant to a fluoroquinolone and an injectable drug) of M. *tuberculosis* was observed in many countries, including Italy (**2**). In order to be in a position to promptly administer the most suitable therapeutic regimen to each patient, there is an urgent need to have available rapid diagnostic tests for the identification of any drug-resistant (DR) strains, so as to be able to begin the therapy as soon as possible (**3**).

In recent years the World Health Organization (WHO) endorsed a number of genotypic and phenotypic assays for the rapid diagnosis of TB and MDR-TB (**4-6**). Among genotypic methods (also referred to as "molecular methods"), commercial Line-Probe Assays using primary cultures or smear-positive specimens yield results in 6-48 hours, but are limited to diagnosis of resistance to rifampicin (INNO-LipA Rif.TB) or rifampicin and isoniazid (GenoType MTBDR-*plus*). A recently endorsed assay based on the assumption that rifampicin resistance in the absence of isoniazid resistance is low (0.5-12%) (**7**) gives results in 2 hours directly from sputum (Xpert MTB/RIF). However, the WHO recommended that patients with rifampicin-resistant strains detected by this assay should receive a treatment for MDR-TB after additional culture-based first-line drug and second-line drug susceptibility test.

Overall, molecular methods can produce fast results but fail to detect 100% of clinically relevant resistance, because of DNA mutations outside the targeted regions and other resistance mechanisms occurring against some first-line drugs (e.g. ethambutol) and second-line injectable drugs (**8**).

The endorsed phenotypic assays (also referred to as "nonmolecular methods"), comprise the conventional solid medium-based proportion and absolute concentration methods, which, due to the slow growth rate of these microorganisms, require more than 3 weeks for drug susceptibility test results, as well as the liquid culture systems, which require 1-2 weeks. The latter include, *inter alia*, the commercial MGIT (Mycobacteria Growth Indicator Tube) 960 system, the non commercial nitrate reductase assay, the resazurin microtiter assay and the Microscopic Observation Drug Susceptibility assay (MODS) assay (**4-6**). In general, liquid culture systems are improvements of existing techniques aimed at decreasing turn-around times for results and intended to increase their availability for laboratories with limited resources.

Unlike genotypic assays, phenotypic assays can provide data on both first-line drugs and second-line drugs, although the reliability of the results varies with the drugs tested and the method used. However, WHO-endorsed phenotypic drug susceptibility test methods are assumed to correctly identify all clinically relevant TB cases.

The most widely used phenotypic susceptibility assays in liquid medium classify *M*. *tuberculosis* isolates as either DR or DS based on their ability to grow in the presence of a critical concentration of the test drug. In mycobacteriology the critical concentration (CC), *i.e.* the antimicrobial susceptibility testing breakpoint, is defined as the lowest concentration of drug that inhibits more than 99% of wild-type *Mycobacterium tuberculosis* strains that have never been exposed to the drug, while at the same time not inhibiting clinical strains of *M*. *tuberculosis* that are considered to be resistant (*i*.*e*., isolated from patients not responding to therapy with the drug). Drug resistance is said to be present when at least 1% of the *M*. *tuberculosis* population grows in the presence of the critical concentration. First-line drugs and second-line drugs critical concentrations are recommended by WHO for each method (**9**). In the practice, the MGIT 960 system, largely used in the world, is considered to be a gold standard for drug susceptibility tests with most first-line drugs and second-line drugs.

Based upon this state of the art and in consideration of the urgency of obtaining rapid drug susceptibility test results in order to begin treating the patient with appropriate anti-TB therapy as soon as possible, new and faster technologies starting from liquid medium-based *M*. *tuberculosis* cultures should be developed. Such new methods should ideally release drug susceptibility test data for both first-line and second line drugs in 1-2 days. A similar approach is expected to integrate reliability of phenotypic assays with fastness of genotypic assays.

### Summary of the invention

In the frame of the studies related to the present invention, a work of Kohanski *et al.* (**10**) concerning the mechanism of cellular death induced by bactericidal antibiotics has been considered, to investigate on possible early markers of drug susceptibility. According to this reference, bactericidal antibiotics with different mechanism of action, after binding to the molecular targets of *Escherichia coli* and *Staphylococcus aureus*, trigger a cascade of events ultimately killing bacteria through the induction of intracellular reactive oxygen species (ROS), such as the hydroxyl radical (^{•}OH). Using the dye hydroxyphenyl fluorescein and flow cytometric enumeration, it was found that aminoglycosides, fluoroquinolones and beta-lactams induce ^{•}OH in *E*. *coli* and *S. aureus* through pathways involving alterations in central and iron metabolisms stimulating the Fenton reaction. This reaction leads to formation of ^{•}OH through the reduction of H₂O₂ by ferrous iron, and causes damages to DNA, proteins and lipids, thereby resulting in bacterial death.

With specific reference to mycobacteria, isoniazid and pyrazinamide were shown, using hydroxyphenyl fluorescein, to induce ^{•}OH formation in *M*. *tuberculosis* as well, leading to release of products that stimulate host cell ROS and autophagy (**11**, **12**). It is also reported in the literature that antibiotic-generated ^{•}OH eradicated *M. tuberculosis* persisters, a small population refractory to antibiotic killing. However, no measurements of ^{•}OH radical were made because the hydroxyphenyl fluorescein dye could not penetrate into the mycobacterial cells (**13**).

In the frame of the studies that led to the present invention it has also been considered that according to several reports rifampicin inhibits *M*. *tuberculosis* transcription by binding to the drug target, *i.e.* the β-subunit of the bacterial RNA-polymerase encoded by the *rpoB* gene (**14**, **15**). It was also observed that ROS were measured in the lysates of the rifampicin-susceptible strain H37Rv (ATCC 27294) but not in the lysates of the isogenic rifampicin-monoresistant mutant strain H37Rv-R (ATCC 35838), having the mutation C531T in the *rpoB* gene and a minimum inhibitory concentration (MIC) of rifampicin >64 µg/ml (**16**, **17**). The above observations demonstrate that, as seen for *E. coli* and *S*. *aureus* (**10**), also in *M. tuberculosis* bactericidal drugs induce intracellular radicals after binding to drug-target.

According to the present invention, it has been considered that besides flow cytometry with fluorescent dyes, the ROS can also be detected by EPR spin trapping, a technique of great specificity and sensitivity for the measurement of free radicals (**18**). Spin trapping reagents react with short-lived radicals, which are subsequently changed to long-lived radicals, called spin-adducts. The advantage of EPR over other techniques is that it allows to detect and identify free radicals through the analysis of spectroscopic characteristics of the spin adduct, and to determine the formation velocity of a radical. The EPR spin trapping technique is a semiquantitative or quantitative method successfully applied to detect various types of radicals from protein radicals to small molecules.

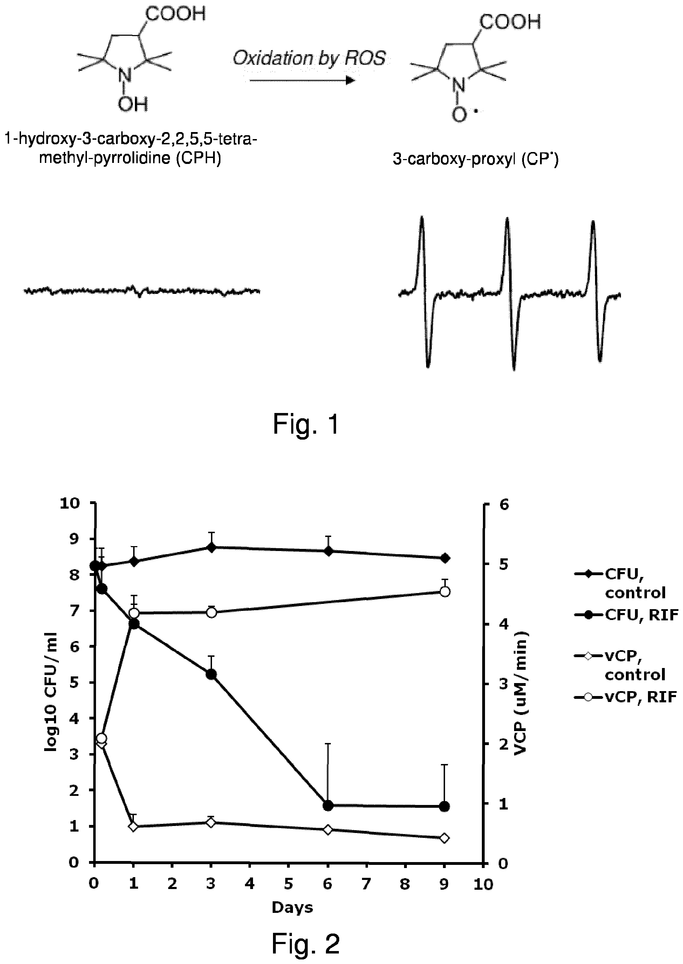

It has now been found, according to the present invention, that when the pan-susceptible reference strain *M. tuberculosis* H37Rv is treated with rifampicin or other bactericidal drugs (*e.g*., moxifloxacin and amikacin), ROS are present in *M. tuberculosis* lysates (*i.e.* inside the bacterial cells) as early as on day 1 post-exposure, and this can be determined by the EPR technique using a suitable spin probe, *e*.*g*., 1-hydroxy-3-carboxy-2,2,5,5-tetramethyl-pyrrolidine (CPH). The latter reacts with unstable radicals to form the stable radical **3-carboxy-2,2,5,5-tetramethyl-1-pyrrolidinyloxyl, generally known as** 3-carboxy-proxyl (CP^{•}), as shown by the following scheme: 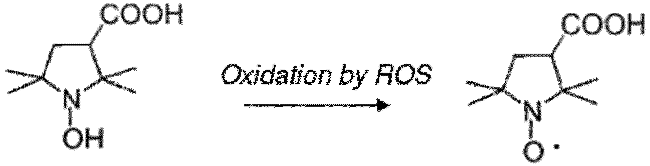 The corresponding EPR spectra, in the absence of exposure to oxidation or after exposure to the ROS present in lysates of a DS *M*. *tuberculosis* strain, are shown in the enclosed **Figure 1**.

As it will be shown in the experimental section herein, the fact that *M*. *tuberculosis* killing by rifampicin induces the formation of radicals which are readily detectable through the EPR technique using CPH as a spin probe was experimentally confirmed in terms of rate of formation of the CP^{•} radical in the presence or in the absence (control) of the drug considered, using the rifampicin-susceptible *M*. *tuberculosis* strain H37Rv mentioned above, and carrying out the EPR analysis in parallel with conventional CFU (colony-forming units) counts, as shown in the enclosed **Figure 2****.**

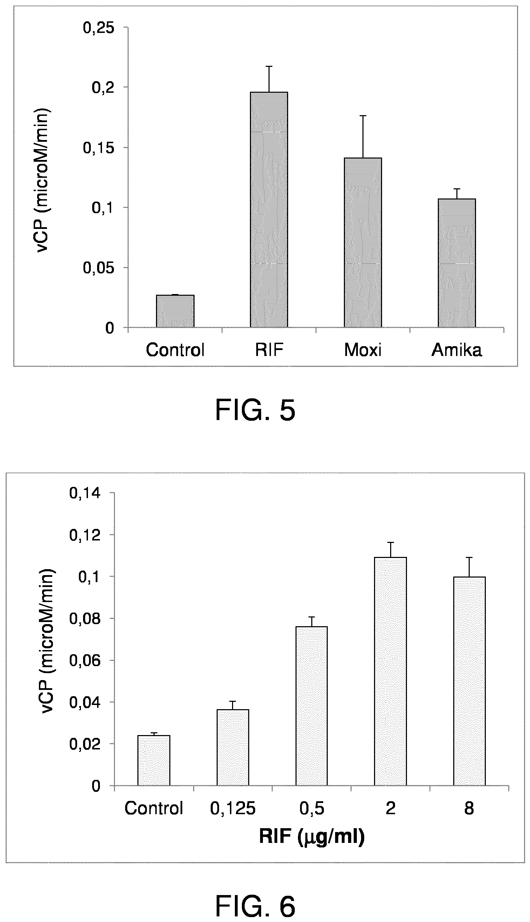

As pointed out above, CP^{•} spin-adducts detectable by EPR have also been shown to be formed upon treatment of the pan-susceptible strain *M*. *tuberculosis* H37Rv with other bactericidal drugs currently used for TB treatment. This is evidenced by the attached **Figure 5****,** in particular for moxifloxacin (a fluoroquinolone) and for amikacin (an injectable drug), both used as second-line drugs for the treatment of the disease.

The relationship between rifampicin and radical production in the pan-susceptible *M*. *tuberculosis* strain H37Rv was further elucidated, according to the present invention, by determining the rate of CP^{•} formation following treatment of tubercle bacilli with rifampicin concentrations ranging from the MIC value (*i*.*e*., 0.125 µg/ml) to the Cₘₐₓ value (maximum drug concentration in serum: 8 µg/ml). These measurements (reported in the diagram of **Figure 6**) showed a clear increase in the rate of CP^{•} formation from MIC to Cₘₐₓ.

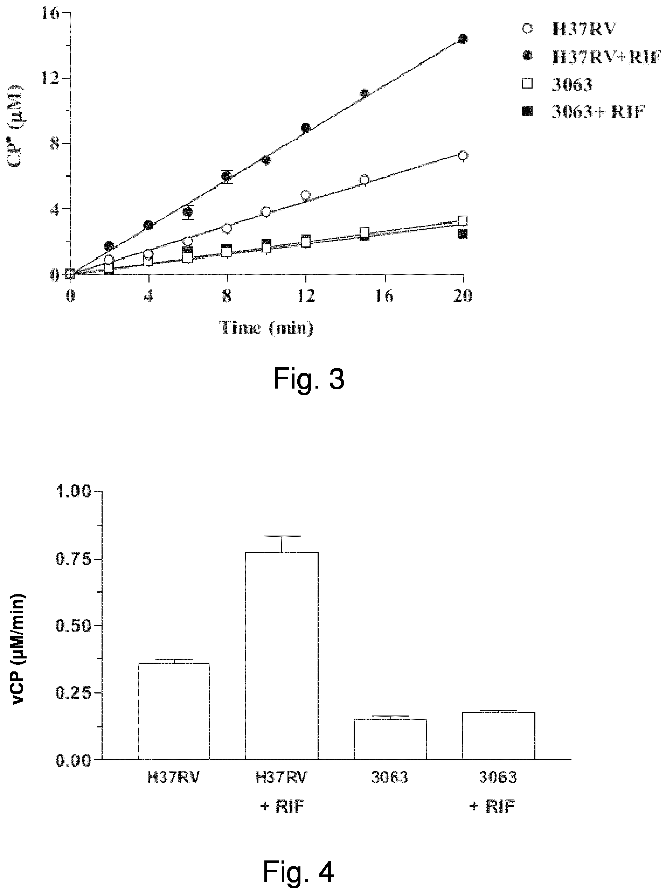

On the other hand, through the experimentation carried out in the frame of the present invention, both rifampicin-susceptible (H37Rv and clinical isolates 5502 and 5050) and rifampicin-resistant (H37Rv-R and clinical isolates 3063, 4164 and 7225) strains were tested. The rifampicin-resistant *M*. *tuberculosis* strains considered, when incubated with the drug, did not show any significant increase in the rate of CP^{•} formation as detected by the EPR technique in comparison with the corresponding untreated control, thereby suggesting that the drug does not bind to its target to an appreciable extent (as shown in the enclosed **Figures 3, 4****,** **7** and **8**).

In view of the foregoing there is proposed, according to the present invention, to perform a *M*. *tuberculosis* antibiogram by applying the EPR technique to measure the rate of formation of radical adducts in mycobacterial lysates after strain exposure to the antibacterial drug under test: according to the foregoing findings, a DS strain is expected to produce radicals at a high rate, while a DR strain is expected to produce radicals at a quite negligible rate.

The diagnostic method according to the invention includes the steps of incubating mycobacteria, after suitable recovery of *M*. *tuberculosis* from clinical isolates and decontamination thereof, with the drug under test for one day, and then, on the second day, lysing the bacterial cells, adding a suitable spin probe and measuring the ROS produced by the action of the drug under test by detecting the formation rate of a spin-adduct (such as CP^{•} in the specific case shown herein). *M. tuberculosis* strains susceptible to the drug under test will produce the sought spin-adduct at a high rate when treated with the drug under test. On the contrary, strains which are not susceptible to the drug will produce the spin-adduct at a quite lower rate, *i.e.* substantially the same formation rate of the adduct in the untreated control. The foregoing result, *i.e.* the rate of formation of a spin-adduct in comparison with that of an untreated control, is readily obtained, according to the invention, by acquiring the EPR spectra of the cell lysates at fixed intervals of time, after addition of the spin probe to the bacterial lysate. The intensity of the first peak of spin-adduct is measured at each time interval and expressed as concentration of radical formed per time unit, with reference to a calibration curve obtained by using standard solutions.

In each case tested, if the rate of increase of the EPR peak intensity is remarkably higher than the rate of increase of the peak intensity in the corresponding control (no drug added) the *M*. *tuberculosis* strain is susceptible to the drug tested, while if such rate is very low and substantially equal to that of the corresponding control, the mycobacterium strain is resistant to the drug being tested.

In some preferred embodiments of the proposed diagnostic method the protocol comprises the following three parts: **a)** standard digestion, decontamination and growth procedure of biological samples from clinical specimen (sputum being the most common TB clinical specimen), which is mostly carried out according to the prior art (**19**); **b)** incubation of the decontaminated biological samples with the drugs under test and *M*. *tuberculosis* lysis procedure; **c)** EPR measurement.

### Brief description of the drawings

The specific features of the present invention, as well as the advantages thereof, will be apparent with reference to the detailed description presented below, and to some experimental results presented by way of example in the accompanying drawings. In the concerned drawings,
**Figure 1** schematically shows the structure of CPH, *i.e.* the spin probe used in a preferred embodiment of the diagnostic method according to the invention, its transformation into a detectable adduct, CP^{•}, upon oxidation by ROS generated by the antibacterial action of a drug under test, specifically rifampicin, as well as the EPR spectra corresponding, respectively, to the starting spin probe and to its adduct with ROS;
**Figure 2** shows the survival *vs*. time of the pan-susceptible strain *M*. *tuberculosis* H37Rv in the absence (control) and in the presence (RIF) of rifampicin (8 µg/ml), as measured in terms of CFU counts (log10 CFU/ml) and in terms of rate of CP^{•} formation (vCP, µM/min) as detected by EPR in the experimentation connected with the invention;
**Figure 3** shows the kinetics of formation of the CP^{•} radical in 20 minutes, in an embodiment of the method according to the invention, in the absence or in the presence of rifampicin, for the pan-susceptible strain *M*. *tuberculosis* H37Rv (ATCC 27294) and for the rifampicin-resistant mutant strain 3063, a clinical isolate;
**Figure 4** shows the rate of CP^{•} formation in the four experimental settings of **Figure 3****,** as calculated from the slope of the corresponding lines, expressed in µM/min;
**Figure 5** shows the experimental increase in the rate of CP^{•} formation after one day of exposure to the Cₘₐₓ of rifampicin (RIF, 8 µg/ml) and, respectively, to the Cₘₐₓ of the second-line drugs moxifloxacin (Moxi, 4 µg/ml) and amikacin (Amika, 8 µg/ml) using the pan-susceptible reference strain *M*. *tuberculosis* H37Rv in the diagnostic method of the invention;
**Figure 6** shows the dose-response to different rifampicin concentrations in the pan-susceptible strain *M. tuberculosis* H37Rv, as detected by the diagnostic method according to the invention;

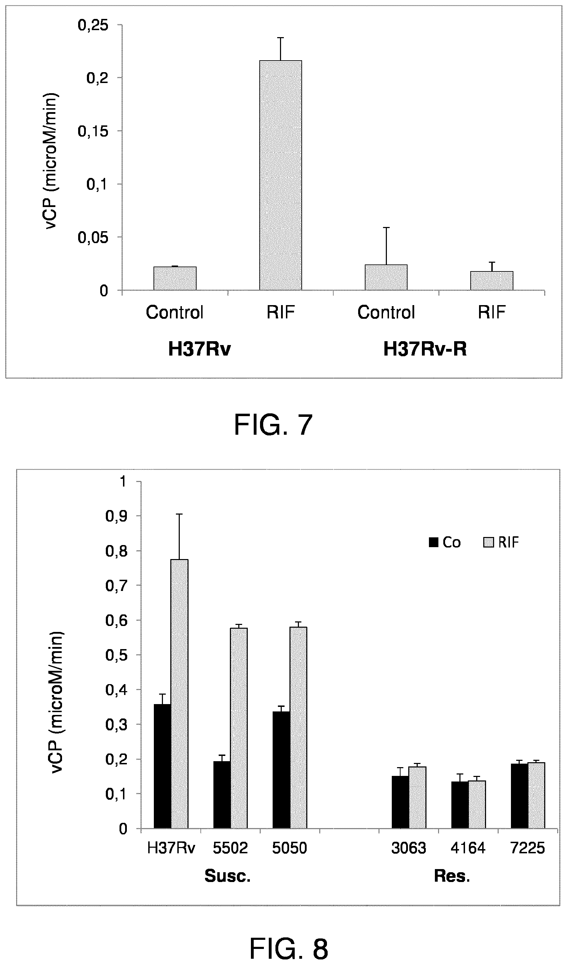

**Figure 7****,** similarly to **Figure 4****,** shows an evidence of rifampicin-induced radicals in *M. tuberculosis* H37Rv but not in the isogenic H37Rv rifampicin-resistant strain (H37Rv-R), as detected by the method of the invention; and
**Figure 8** shows a demonstration of susceptibility/resistance to rifampicin in six *M*. *tuberculosis* strains (three rifampicin-susceptible: H37Rv and clinical isolates 5502 and 5050, and three rifampicin-resistant: clinical isolates 3063, 4164 and 7225), as detected by applying the method of the invention.

### Detailed description of the invention

Thus, the present invention specifically provides a method for determining susceptibility of *M. tuberculosis* to one or more antibacterial drugs proposed for therapy of a TB patient, said method comprising the following steps:
A) subjecting a clinical isolate from said patient to conventional preliminary procedures for decontamination of said clinical isolate and incubation of the resulting *M. tuberculosis* in liquid culture medium until a visible growth is observed;
B) adding to a first portion of liquid culture resulting from the previous step a selected antibacterial drug while keeping a corresponding second portion of liquid culture as a control,
   incubating at 37°C for a selected time and then collecting the *M*. *tuberculosis* cells from the respective cultures, and lysing said cells;
   this operation being repeated for each antibacterial drug proposed for therapy of said patient;
C) adding to the respective portions of cell lysates obtained from the previous step a spin probe selected from the group consisting of: 1-hydroxy-3-carboxy-2,2,5,5-tetramethylpyrrolidine (CPH), 1-hydroxy-4-methoxy-2,2,6,6-tetramethylpiperidine (TMH), 1-hydroxy-3-methoxycar-bonyl-2,2,5,5-tetramethylpyrrolidine (CMH) and other spin probes used for detection of ROS in EPR techniques (such as, *e*.*g*., other suitable cyclic hydroxylamines,
   acquiring EPR spectra from each respective portion of cell lysates obtained upon addition of said spin probes at fixed time intervals starting from 1-5 minutes after the respective addition, for a total time of from 10 to 60 minutes, measuring the intensity of the first peak of the respective spectrum at each time interval to obtain the growth rate of said intensity in time and comparing said growth rate with the growth rate obtained for the corresponding control;
   wherein a growth rate substantially higher than in the corresponding control is an indication of susceptibility of the *M*. *tuberculosis* present in the clinical isolate to the antibacterial drug tested, while a low growth rate, substantially equal to the growth rate in the corresponding control is an indication of resistance of said *M. tuberculosis* to the antibacterial drug tested.

According to some specific embodiments of the invention, said fixed time intervals in step C) can be from 1 to 5 minutes, and preferably they are of 2 minutes. Preferably, said fixed time intervals start from 2 minutes after the respective addition. According to some preferred embodiments, the total time in step C) may be comprised between 10 and 30 minutes, and more preferably it is of 20 minutes.

With reference to the step B) of incubation of the decontaminated *M*. *tuberculosis* cells with the antibacterial drug under test, said selected time of incubation may range from 8 to 36 hours, and preferably it is of 24 hours.

A general protocol of the diagnostic method according to the present invention may be summarized as follows:
**a) Decontamination and growth procedure**
   Optimal recovery of *M*. *tuberculosis* from clinical specimens (mostly consisting of sputum or bronchial lavages) known to be contaminated requires liquefying the organic debris (digestion) and eliminating non-mycobacterial contaminating organisms (decontamination). The procedure most used worldwide is the N-acetyl-L-cysteine (NALC) - NaOH method. After digestion-decontamination, mycobacterial cells are inoculated in liquid culture media such as Dubos Tween-albumin (DTA) broth or Middlebrook 7H9 broth, and incubated at 37°C until a visible growth (logarithmic phase) is observed.
**b) Culture incubation with drugs and cell lysis**
   First- and second-line anti-TB drugs are added to liquid cultures and incubated for various times at 37°C. Mycobacteria are collected by centrifugation, re-suspended to a suitable cell density, and then lysed using any common method, such as (**20**):
   1. Mechanical disruption, for example by a Mini-Beadbeater apparatus in the presence of suitable beads, using several cycles of 50-s each at maximum speed with a 60-s rest on ice between pulses;
   2. Enzymatic digestion, for example by combined chloroform-methanol exposure followed by lysozyme incubation at 37°C and final treatment with sodium dodecyl sulphate (SDS)-proteinase K at 55°C;
   3. Chemical lysis, for example by exposure to cetyltrimethylammonium bromide (CTAB), heating at 80°C, incubation with lysozyme at 37°C, followed by treatment with SDS-Proteinase K at 65°C and with CTAB-NaCl at 65 °C;
   4. Heat lysis, for example by incubation at 95°C for 20-30 minutes, and then water sonication for 10-30 minutes.
**c) EPR measurements**
   A variable volume of the cell lysate is used for EPR measurements. Protein content is normalized by a quantitation method. Different spin probes can be used, including the following ones:
   1) : 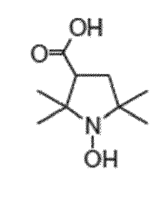
   2) 1-Hydroxy-4-methoxy-2,2,6,6-tetramethylpiperidine (TMH): 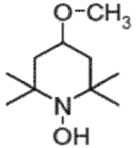
   3) 1-Hydroxy-3-methoxycarbonyl-2,2,5,5-tetramethylpyrrolidine (CMH): 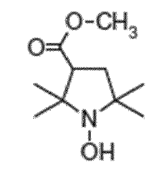
   4) Other hydroxylamines, specifically cyclic hydroxylamines, suitable for use as spin traps or spin probes in EPR techniques.

   After addition of the spin probe to the cell lysate, EPR spectra are acquired at intervals of from 1 to 5 min for a time of 10-30 min, as a function of the spin probe used. The chosen acquisition time guarantees the linearity of the spin probe reaction with ROS formed in the samples. The EPR spectra so acquired are used to measure the intensity of the first peak of spin-adduct at each time interval, and the data obtained are referred to a calibration curve obtained by using standard solutions, to give the rate of formation of the spin adduct.

As pointed out before, each of said antibacterial drugs of step B) may be selected from the general group of antibacterial agents already in use for the treatment of TB, including the so-called first line drugs rifampicin, isoniazid, ethambutol and pyrazinamide and the second-line drugs, *i.e.* ofloxacin and moxifloxacin (fluoroquinolones), amikacin, kanamycin and capreomycin (injectable drugs) and linezolid, clofazimine and meropenem (other drugs). In accordance with some preferred embodiments thereof, the method of the invention has been applied to perform an antibiogram with rifampicin, moxifloxacin and amikacin.

As it appears from the foregoing, the preferred spin probe which has been successfully used to perform rapid and reliable antibiograms using the three antibacterials mentioned above is CPH. It should be noted that since the research of spin probes able to better detect ROS formation in biological samples is continuously in progress, this protocol could be improved by using different hydroxylamines, or in general other spin probes, more sensitive for ROS detection or specific for the oxidant formed. This would allow to reduce the spin probe concentration, the acquisition time and/or the amount of *M*. *tuberculosis* lysates proposed in this protocol.

The diagnostic method according to the invention provides a rapid detection of resistance of *M*. *tuberculosis* to anti-TB drugs. Instead of obtaining an antibiogram in 7-14 days as it is presently the case when using the MGIT 960 system, the proposed method offers a response in 2 days, thus allowing a more rapid diagnosis of drug resistance and, consequently, a more immediate and effective TB therapy.

### Examples and experimental results

Some experimental results, obtained with the application of some specific embodiments of the diagnostic method of the present invention, are given by way of example in the following.

### Preliminary investigation results

As pointed out in the foregoing, the initial findings of the present invention were confirmed by a series of experimental evidences, based on the use of the spin probe 1-hydroxy-3-carboxy-2,2,5,5-tetramethyl-pyrrolidine, *i.e.* CPH. As shown in shown in **Figures 1** and **2**, CPH has been shown to react with the unstable ROS generated by the action of an antibacterial drug on a susceptible bacterial strain to form a stable spin adduct, *i.e.* the 3-carboxy-proxyl radical (CP^{•}), which can be easily detected by EPR spectroscopy.

Using CPH as a spin probe on bacterial lysates from strains susceptible to a given antibacterial agent, after treatment of said strains with the concerned agent, resulted in a rate of formation of the CP^{•} radical, as detected from the EPR spectra, much higher than the corresponding rate of the untreated control. To this regard, **Figure 2** shows *M*. *tuberculosis* survival (log10 CFU/ml) in terms of CFU counts and rate of CP^{•} formation (µM/min) after 1 day and 3, 6 and 9 days of treatment with rifampicin. Means and standard deviations (SD) from three experiments are shown.

In order to compare the performance of the diagnostic procedure proposed in cases of DS strains and in cases of DR strains the kinetics of formation of CP^{•} in the absence or in the presence of rifampicin was investigated for the pan-susceptible strain *M. tuberculosis* H37Rv and for a rifampicin-resistant mutant strain, referred to as strain 3063, a clinical isolate. For the two strains, as well as for the corresponding controls, the intensities of the first EPR peak, measured in arbitrary units, were converted in µMol of CP^{•} and plotted vs. time, as shown in **Figure 3****.**

The corresponding rate of CP^{•} formation was then calculated from the slope of the corresponding lines of **Figure 3** and expressed in µM/min, thereby obtaining the histogram of **Figure 4****.** As shown by this figure, all samples treated with drugs induced an increase in the rate of CP^{•} formation (µM/min), while the H37Rv not incubated with the drug (Control) produces CP^{•} radicals at quite lower rate than the H37Rv incubated with the drug, in the case of the resistant strain 3063 the respective rates of CP^{•} formation of treated sample and control are practically the same, thereby showing that no activity is being exerted by the tested drug on *M. tuberculosis* strain 3063.

The method under investigation was then tested to evaluate the response of the pan-susceptible *M*. *tuberculosis* H37Rv to rifampicin as a first-line drug, and to other bactericidal drugs, *e*.*g*. moxifloxacin and amikacin. As it is shown in **Figure 5****,** showing the increase in the rate of CP^{•} formation, obtained by EPR measurements, after one day of exposure to the Cₘₐₓ of rifampicin (RIF, 8 µg/ml) and, respectively, to the Cₘₐₓ of moxifloxacin (Moxi, 4 µg/ml) and amikacin (Amika, 8 µg/ml), ROS were found to be produced in all samples incubated with these drugs at a quite high rate, as compared to H37Rv not incubated with the drug (Control). i

To further investigate the relationship between rifampicin and radical production in *M. tuberculosis* H37Rv, the rate of CP^{•} formation following treatment of tubercle bacilli with rifampicin concentrations ranging from the MIC value (0.125 µg/ml) to the Cₘₐₓ value (maximum drug concentration in serum: 8 µg/ml) was determined. The pan-susceptible strain *M. tuberculosis* H37Rv was incubated in the presence of 0.125, 0.5, 2.0 and 8.0 µg/ml rifampicin for 24 hours. As shown in **Figure 6****,** the rate of CP^{•} formation increased with drug concentration, with the highest values being observed with 2 and 8 µg/ml of rifampicin.

The effectiveness of the proposed method based on EPR spin-trapping in discriminating between DS and DR *M*. *tuberculosis* strains was further evaluated on the pan-susceptible *M*. *tuberculosis* strain H37Rv in comparison with the isogenic rifampicin-resistant strain H37Rv-R. H37Rv-R carries a mutation in the target gene *rpoB* (β-subunit of RNA polymerase), which confers resistance to rifampicin. Also in this case, as shown in **Figure 7****,** the rifampicin-susceptible strain showed a strong increase in the rate of CP^{•} formation after incubation with rifampicin, compared to control bacteria in absence of the drug. In contrast, the rifampicin -resistant strain H37Rv RIF-R failed to show any increase the rate of CP^{•} formation.

To further confirm the effectiveness of the proposed diagnostic method in discriminating between DS and DR bacterial strains, three rifampicin-susceptible strains (H37Rv and two clinical isolates, *i.e.* strains 5502 and 5050) and three rifampicin-resistant clinical isolates, as determined by the MGIT 960 system (*i.e.* strains 3063, 4164 and 7225, all carrying mutations in the *rpoB* gene), were treated with 8 µg/ml (Cₘₐₓ) of rifampicin for 24 hours, lysed with zirconia beads as described herein in the following section and then tested by EPR on the second working day for determining the rate of CP^{•} formation.

As shown in **Figure 8****,** the EPR procedure showed that all rifampicin-susceptible strains induced an increase in radical production in the presence of rifampicin as compared to strains not incubated with the drug (Control, Co), whereas no increase in radical induction was observed in rifampicin-resistant strains. The proposed protocol was performed in 2 working days.

### Detailed protocol of a preferred example

In the experimentation reported herein two different EPR spectrometers were used, a highly performant EPR instrument used in laboratory research (ECS 106, Bruker, Rheinstetten, Germany), which proved to be more sensitive in radical species detection, and a smaller benchtop instrument (Bruker e-scan), which is more suitable for quality control applications, as well as for medical and pharmaceutical applications. The latter proved to be effective as well, despite its lower sensitivity.

The detailed protocol applied is as follows:
**a) Standard digestion-decontamination-growth procedure from sputum** Clinical isolates (*i*.*e*., sputa) collected from patients are decontaminated from microorganisms other than mycobacteria, and inoculated in liquid medium to grow a sufficient number of cells to detect changes in intracellular radicals.
   1. Add an equal volume of N-acetyl-L-cysteine (NALC) containing 2% NaOH to sputum samples (maximum 10 ml) in a 50-ml tube. Agitate the tube on a vortex mixer and let the tube stand for 15 min at room temperature.
   2. Dilute mixtures up to 50 ml with 0.067 M phosphate buffer to reduce digestion-decontaminating effects of NALC-NaOH.
   3. Spin tubes at 3000 x *g* for 15 min.
   4. Suspend sediments in 1 ml of distilled water (DW) or 0.2% bovine serum albumin fraction V. Prepare a smear of each sediment, fix by heat or methanol and stain by the Ziehl-Neelsen method to check for the presence of acid-fast bacilli (AFB, *i.e.* mycobacteria).
   5. Make a 1:10 dilution of sediments with DW and inoculate 0.1 ml onto solid media (Löwenstein-Jensen and Middlebrook 7H10) to visualize AFB colonies and exclude contaminations. Identify M. *tuberculosis* by routine (molecular preferred) methods.
   6. Inoculate also a liquid medium (DTA broth), and incubate at 37°C up to optical density (OD) at 600 nm of about 0.4, for EPR-antibiogram.
**b) Culture incubation with drugs and cell lysis**
   Once pretreated as in the previous section, *M. tuberculosis* is incubated in absence or presence of the chosen drug concentration prior to cell lysis, obtained by cell disruption.
   1. Add first- and second-line anti-TB drug to DTA-cultures of M. *tuberculosis* [at concentrations to be defined in comparative studies using EPR and worldwide-used methods **(9)**].
   2. Incubate cultures at 37°C for 24 h.
   3. Centrifuge a portion of cultures at 1600 x *g* for 30 min at room temperature
   4. Discard supernatants.
   5. Suspend sediments in phosphate buffered saline (PBS).
   6. Centrifuge at 1600 x *g* for 30 min at room temperature.
   7. Discard supernatants.
   8. Suspend sediments in 1 ml of PBS.
   9. Transfer samples in 1.5 ml screw-cap tubes containing 0.1 mm zirconia/silica beads. Lyse *M*. *tuberculosis* cells at maximum speed through 6 cycles of 50-s each with a 60-s rest on ice between pulses using a Mini-Beadbeater apparatus.
   10. Spin lysates at maximum speed for 5 min.
   11. Filter supernatants through 0.22 µm filters to sterilize extracts. Filtering could be substituted by heating lysates at 80°C for 1 hour to kill remaining live mycobacteria.
**c) EPR measurements**
   The sterilized extracts undergo analysis and quantitation of intracellular radical molecules present in bacterial lysates incubated in the absence or presence of the drug using an EPR instrument.
   1. Use 0.1 ml of each sample for EPR measurements. Bacterial samples are normalized for protein content on the basis of OD₂₈₀ₙₘ readings by adding an aliquot of each extract into 0.1 ml final volumes.
   2. Add 5 µl of the spin probe (CPH, stock solution: 10 mM, in degassed-Chelex 100-treated PBS; final concentration 0.5 mM).
   3. Acquire EPR spectra at intervals of 2 min for 20 min, with the first spectrum being acquired exactly 2 min after spin probe addition.
   4. Measure the intensity of the first peak of the corresponding nitroxide 3-carboxy-proxyl radical (CP^{•}) formed at the chosen time, and express as µM radical formed/min referring to a calibration curve obtained by using standard solutions of 3-carboxy-proxyl radical.

It should be noted that the standard procedure and b) steps need to be performed in a Biosafety Level 3 room.

With reference to item b)1 above (concentrations of the drugs under test), it will be appreciated that a dose-response for each first-line drug and second-line drug should be performed to determine the optimal drug concentration (see **Fig. 6** as an example for rifampicin). To this purpose, a panel of DS and DR M. *tuberculosis* reference strains provided by WHO for drug susceptibility testing quality assurance can be incubated with each first-line drug and second-line drug for 24 hours in the presence of multiples of MGIT critical concentrations (MGIT-CC) (**9**) as follows: 0.125xCC, 0.25xCC, 0.5xCC, 1xCC, 2xCC, 4xCC, 8xCC and 0xCC (drug-untreated control). After centrifugation, lysis, filtration and spin probe addition, the rate of CP^{•} formation can be determined on day 2 by EPR as described above.

For each DS isolate, the difference in the rate of radical formation between drug-treated and drug-untreated cells can be plotted against drug concentrations. The EPR critical concentration (EPR-CC) can be defined as the lowest drug concentration inducing the highest radical level in the DS strains tested. This concentration can be used as a cut-off to differentiate DS-from DR-strains, in comparison with WHO results (gold standard). No DR-strain should generate radicals at levels significantly higher than baseline untreated controls (see **Fig. 6** as an example).

EPR-antibiograms of a large panel of strains can be compared with WHO results and statistical parameters can be calculated, including sensitivity (ability to detect true resistance), specificity (ability to detect true susceptibility), predictive values for resistance (PVR, *i*.*e*., the rate of true resistance to total resistance) and for susceptibility (PVS, *i*.*e*., the rate of true susceptibility to total susceptibility), efficiency (ratio between the number of correct results and the total number of results), and reproducibility (intra-laboratory agreement between duplicate cultures).

Besides being quite faster than known phenotypic assays, the diagnostic method according to the invention is more reliable than rapid molecular tests, as it differentiates DS from DR strains through the detection of radical species responsible for the bacterial cell death, while rapid molecular tests do not cover new mutations in target genes. Further, the proposed diagnostic method can be easily applicable to all bactericidal drugs.

In addition, while the main application of the present invention consists in the determination of drug resistance in *M. tuberculosis,* potentially the proposed method can be extended to other slowly growing bacterial species (e.g. nontuberculous mycobacteria and *Nocardia* spp.).

The present invention has been disclosed with particular reference to some specific embodiments thereof, but it should be understood that modifications and changes may be made by the persons skilled in the art without departing from the scope of the invention as defined in the appended claims.

### References

1. World Health Organization. Global tuberculosis report 2012. WHO/HTM/TB/2012.6.
2. Drug-resistant tuberculosis among foreign-born persons in Italy. Fattorini L, Mustazzolu A, Piccaro G, Pardini M, Filippini P, Giannoni F, Migliori GB, Sotgiu G, Borroni E, Cirillo DM; Italian Multicentre Study on Resistance to Antituberculosis Drugs Group. Eur Respir J. 2012; 40:497-500.
3. Giannoni F, Iona E, Sementilli F, Brunori L, Pardini M, Migliori GB, Orefici G, Fattorini L. Evaluation of a new line probe assay for rapid identification of gyrA mutations in Mycobacterium tuberculosis. Antimicrob Agents Chemother. 2005; 49:2928-33.
4. Wilson ML. Recent advances in the laboratory detection of Mycobacterium tuberculosis complex and drug resistance. Clin Infect Dis. 2011; 52:1350-5.
5. Moore DA, Shah NS. Alternative methods of diagnosing drug resistance--what can they do for me? J Infect Dis. 2011; 204 Suppl.4: S1110-9.
6. Rigouts L, Gumusboga M, de Rijk WB, Nduwamahoro E, Uwizeye C, de Jong B, Van Deun A. Rifampin resistance missed in automated liquid culture system for Mycobacterium tuberculosis isolates with specific rpoB mutations. J Clin Microbiol. 2013; 51:2641-5.
7. Lawn SD, Mwaba P, Bates M, Piatek A, Alexander H, Marais BJ, Cuevas LE, McHugh TD, Zijenah L, Kapata N, Abubakar I, McNerney R, Hoelscher M, Memish ZA, Migliori GB, Kim P, Maeurer M, Schito M, Zumla A. Advances in tuberculosis diagnostics: the Xpert MTB/RIF assay and future prospects for a point-of-care test. Lancet Infect Dis. 2013; 13:349-61.
8. Miotto P, Cabibbe AM, Mantegani P, Borroni E, Fattorini L, Tortoli E, Migliori GB, Cirillo DM. GenoType MTBDRsl performance on clinical samples with diverse genetic background. Eur Respir J. 2012; 40:690-8.
9. Clinical and Laboratory Standards Institute. 2011. Susceptibility testing of Mycobacteria, Nocardiae, and Other Aerobic Actinomycetes; Approved Standard - Second Edition. M24-02 vol 31. No. 5.
10. Kohanski MA, Dwyer DJ, Hayete B, Lawrence CA, Collins JJ. A common mechanism of cellular death induced by bactericidal antibiotics. Cell. 2007; 130:797-810.
11. Kim JJ, Lee HM, Shin DM, Kim W, Yuk JM, Jin HS, Lee SH, Cha GH, Kim JM, Lee ZW, Shin SJ, Yoo H, Park YK, Park JB, Chung J, Yoshimori T, Jo EK. 2012. Host cell autophagy activated by antibiotics is required for their effective antimycobacterial drug action. Cell Host Microbe. 11:457-68.
12. Goletti D, Petruccioli E, Romagnoli A, Piacentini M, Fimia GM. 2013. Autophagy in Mycobacterium tuberculosis infection: a passepartout to flush the intruder out? CytokineGrowth Factor Rev. 24:335-43.
13. Grant SS, Kaufmann BB, Chand NS, Haseley N, Hung DT. Eradication of bacterial persisters with antibiotic-generated hydroxyl radicals. Proc Natl Acad Sci U S A. 2012; 109:12147-52.
14. Sacchettini JC, Rubin EJ, Freundlich JS. 2008. Drugs versus bugs: in pursuit of the persistent predator Mycobacterium tuberculosis. Nat Rev Microbiol. 6:41-52.
15. Telenti A, Imboden P, Marchesi F, Lowrie D, Cole S, Colston MJ, Matter L, Schopfer K, Bodmer T. 1993. Detection of rifampicin-resistance mutations in Mycobacterium tuberculosis. Lancet. 341:647-50.
16. Garcia L, Alonso-Sanz M, Rebollo MJ, Tercero JC, Chaves F. 2001. Mutations in the rpoB gene of rifampin-resistant Mycobacterium tuberculosis isolates in Spain and their rapid detection by PCR-enzyme-linked immunosorbent assay. J Clin Microbiol. 39:1813-8.
17. Fattorini L, Iona E, Ricci ML, Thoresen OF, Orrù G, Oggioni MR, Tortoli E, Piersimoni C, Chiaradonna P, Tronci M, Pozzi G, Orefici G. 1999. Activity of 16 antimicrobial agents against drug-resistant strains of Mycobacterium tuberculosis. Microb Drug Resist. 5:265-70.
18. Kohno M. Applications of electron spin resonance spectrometry for reactive oxygen species and reactive nitrogen species research. J Clin Biochem Nutr. 2010; 47:1-11.
19. De Waard J.H., Robledo J. "Conventional Diagnostic Methods", Chapter 12 in "Tuberculosis 2007: from basic science to patient care", Palomino J.C., Leão S.C., Ritacco V. editors.
20. Okwumabua O, Shull E, O'Connor M, Moua TV, Danz T, Strelow K. "Comparison of three methods for extraction of Mycobacterium avium subspecies paratuberculosis DNA for polymerase chain reaction from broth-based culture systems". J Vet Diagn Invest. 2010 Jan; 22(1):67-9.

## Claims

1. A method for determining susceptibility of *Mycobacterium tuberculosis* to one or more antibacterial drugs proposed for therapy of a tuberculosis patient, said method comprising the following steps:
A) subjecting a clinical isolate from said patient to conventional preliminary procedures for decontamination of said clinical isolate and incubation of the resulting *M. tuberculosis* in liquid culture medium until a visible growth is observed;
B) adding to a first portion of liquid culture resulting from the previous step a selected antibacterial drug while keeping a corresponding second portion of liquid culture as a control,
incubating at 37°C for a selected time and then collecting the *M*. *tuberculosis cells* from the respective cultures, and lysing said cells;
this operation being repeated for each antibacterial drug proposed for therapy of said patient;
C) adding to the respective portions of cell lysates obtained from the previous step a spin probe selected from the group consisting of: 1-hydroxy-3-carboxy-2,2,5,5-tetramethylpyrrolidine (CPH), 1-hydroxy-4-methoxy-2,2,6,6-tetramethylpiperidine (TMH), 1-hydroxy-3-methoxycar-bonyl-2,2,5,5-tetramethylpyrrolidine (CMH) and other spin probes used for detection of ROS in EPR (electron paramagnetic resonance) techniques;
acquiring EPR spectra from each respective portion of cell lysates obtained upon addition of said spin probes at fixed time intervals starting from 1-5 minutes after the respective addition, for a total time of from 10 to 60 minutes, measuring the intensity of the first peak of the respective spectrum at each time interval to obtain the growth rate of said intensity in time and comparing said growth rate with the growth rate obtained for the corresponding control;
wherein a growth rate substantially higher than in the corresponding control is an indication of susceptibility of the *M*. *tuberculosis* present in the clinical isolate to the antibacterial drug tested, while a low growth rate, substantially equal to the growth rate in the corresponding control is an indication of resistance of said *M. tuberculosis* to the antibacterial drug tested.

2. A method according to claim 1, wherein said fixed time intervals in step C) are of from 1 to 5 minutes.

3. A method according to claims 1 or 2, wherein said fixed time intervals start from 2 minutes after the respective addition.

4. A method according to claim 3, wherein said fixed time intervals in step C) are of 2 minutes.

5. A method according to claim 4, wherein said total time in step C) is from 10 to 30 minutes, and preferably it is of 20 minutes.

6. A method according to claims 1 or 5 , wherein said selected time of incubation in step B) is from 8 to 36 hours, and preferably it is of 24 hours.

7. A method according to claim 6, wherein each of said antibacterial drug of step B) is selected from the group consisting of: rifampicin, isoniazid, ethambutol, pyrazinamide, ofloxacin, moxifloxacin, amikacin, kanamycin, capreomycin, linezolid, clofazimine and meropenem.

8. A method according to claim 7, wherein said proposed antibacterial drugs are rifampicin, moxifloxacin and amikacin.

9. A method according to claims 1 or 8, wherein said spin probe is 1-hydroxy-3-carboxy-2,2,5,5-tetramethylpyrrolidine (CPH).

10. A method according to claims 1 or 7, wherein said other spin probes used for detection of ROS in EPR techniques are selected from the group of cyclic hydroxylamines.

## Patentansprüche

1. Verfahren zur Bestimmung der Suszeptibilität von *Mycobacterium tuberculosis* für einen oder mehrere antibakterielle(n) Wirkstoff(e), der bzw. die für die Therapie eines Tuberkulose-Patienten vorgeschlagen wird/werden, wobei das Verfahren die folgenden Schritte umfasst:
A) Unterwerfen eines klinischen Isolats des Patienten herkömmlichen vorbereitenden Prozeduren zur Dekontaminierung des klinischen Isolats und Inkubationn der resultierenden *M*. *tuberculosis* in Flüssigkulturmedium, bis ein sichtbares Wachstum beobachtet wird;
B) Zugeben eines ausgewählten antibakteriellen Wirkstoffs zu einem ersten Anteil der aus dem vorhergehenden Schritt resultierenden Flüssigkultur, während ein entsprechender zweiter Anteil der Flüssigkultur als Kontrolle behalten wird,
Inkubieren bei 37°C für eine ausgewählte Zeit und anschließendes Gewinnen der *M*. *tuberculosis-Zellen* aus den jeweiligen Kulturen, sowie Lysieren der Zellen; wobei dieser Vorgang für jeden für die Therapie des Patienten vorgeschlagenen antibakteriellen Wirkstoff wiederholt wird;
C) Zugeben einer Spin-Sonde, welche aus der Gruppe ausgewählt ist, die aus 1-Hydroxy-3-carboxy-2,2,5,5-tetramethylpyrrolidin (CPH), 1-Hydroxy-4-methoxy-2,2,6,6-tetramethylpiperidin (TMH), 1-Hydroxy-3-methoxycarbonyl-2,2,5,5-tetramethylpyrrolidin (CMH) und anderen Spin-Sonden, die zum Nachweis von ROS in EPR (paramagnetischen Elektronenresonanz)-Techniken verwendet werden, besteht, zu den jeweiligen Anteilen von Zelllysaten, die aus dem vorhergehenden Schritt erhalten wurden;
Erstellen der EPR-Spektren von jedem jeweiligen Anteil der Zelllysate, die nach Zugabe der Spin-Sonden erhalten wurden, in festgelegten Zeitintervallen, beginnend mit 1-5 Minuten nach der jeweiligen Zugabe, für einen Gesamtzeitraum von 10 bis 60 Minuten, Messen der Intensität des ersten Peaks des jeweiligen Spektrums zu jedem Zeitintervall, um die Wachstumsrate der Intensität mit der Zeit zu erhalten, und Vergleichen dieser Wachstumsrate mit der Wachstumsrate, die für die entsprechende Kontrolle erhalten wird;
wobei eine wesentlich höhere Wachstumsrate als in der entsprechenden Kontrolle Suszeptibilität der im klinischen Isolat vorhandenen *M*. *tuberculosis* für den getesteten antibakteriellen Wirkstoff anzeigt, während eine niedrige Wachstumsrate, im Wesentlichen gleich der Wachstumsrate in der entsprechenden Kontrolle, Resistenz der *M*. *tuberculosis* gegenüber dem getesteten antibakteriellen Wirkstoff anzeigt.

2. Verfahren nach Anspruch 1, wobei die festgelegten Zeitintervalle in Schritt C) 1 bis 5 Minuten betragen.

3. Verfahren nach Anspruch 1 oder 2, wobei die festgelegten Zeitintervalle 2 Minuten nach der jeweiligen Zugabe beginnen.

4. Verfahren nach Anspruch 3, wobei die festgelegten Zeitintervalle in Schritt C) 2 Minuten betragen.

5. Verfahren nach Anspruch 4, wobei die Gesamtzeit in Schritt C) 10 bis 30 Minuten beträgt und vorzugsweise 20 Minuten beträgt.

6. Verfahren nach den Ansprüchen 1 oder 5, wobei die ausgewählte Inkubationszeit in Schritt B) 8 bis 36 Stunden beträgt und vorzugsweise 24 Stunden beträgt.

7. Verfahren nach Anspruch 6, wobei jeder der antibakteriellen Wirkstoffe von Schritt B) aus der Gruppe ausgewählt ist, welche aus Rifampicin, Isoniazid, Ethambutol, Pyrazinamid, Ofloxacin, Moxifloxacin, Amikacin, Kanamycin, Capreomycin, Linezolid, Clofazimin und Meropenem besteht.

8. Verfahren nach Anspruch 7, wobei die vorgeschlagenen antibakteriellen Wirkstoffe Rifampicin, Moxifloxacin und Amikacin sind.

9. Verfahren nach Anspruch 1 oder 8, wobei die Spin-Sonde 1-Hydroxy-3-carboxy-2,2,5,5-tetramethylpyrrolidin (CPH) ist.

10. Verfahren nach Anspruch 1 oder 7, wobei die anderen Spin-Sonden, die für den Nachweis von ROS in EPR-Techniken verwendet werden, aus der Gruppe von cyclischen Hydroxylaminen ausgewählt sind.

## Revendications

1. Procédé pour déterminer la sensibilité de la souche *Mycobacterium tuberculosis* à un ou plusieurs médicaments antibactériens proposés pour la thérapie d'un patient atteint de tuberculose, ledit procédé comprenant les étapes suivantes consistant à :
A) soumettre un isolat clinique venant dudit patient à des procédures préliminaires classiques pour la décontamination dudit isolat clinique et l'incubation de la souche *M. tuberculosis* obtenue dans un milieu de culture liquide jusqu'à ce qu'une croissance visible soit observée ;
B) ajouter à une première partie de culture liquide résultant de l'étape précédente un médicament antibactérien choisi tout en maintenant une seconde partie correspondante de culture liquide comme témoin,
incuber à 37 °C pendant une période de temps choisie et recueillir ensuite les *cellules de M. tuberculosis* des cultures respectives et lyser lesdites cellules ; cette opération étant répétée pour chaque médicament antibactérien proposé pour la thérapie dudit patient ;
C) ajouter aux parties respectives de lysats cellulaires obtenus à l'étape précédente une sonde de spin choisie dans le groupe constitué de la 1-hydroxy-3-carboxy-2,2,5,5-tétraméthylpyrrolidine (CPH), de la 1-hydroxy-4-méthoxy-2,2,6,6-tétraméthylpipéridine (TMH), de la 1-hydroxy-3-méthoxycarbonyl-2,2,5,5-tétraméthylpyrrolidine (CMH) et d'autres sondes de spin utilisées pour la détection du ROS dans des techniques EPR (résonance paramagnétique électronique) ;
acquérir des spectres EPR pour chaque partie respective de lysats cellulaires obtenus lors de l'addition desdites sondes de spin à intervalles de temps fixes en partant de 1 à 5 minutes après l'addition respective pendant une période totale de 10 à 60 minutes, mesurer l'intensité du premier pic du spectre respectif à chaque intervalle de temps pour obtenir le taux de croissance de ladite intensité dans le temps et comparer ledit taux de croissance au taux de croissance obtenu pour le témoin correspondant ;
dans lequel un taux de croissance sensiblement plus élevé que dans le témoin correspondant est une indication de sensibilité de la souche *M. tuberculosis* présente dans l'isolat clinique au médicament antibactérien testé, tandis qu'un taux de croissance bas sensiblement égal au taux de croissance dans le témoin correspondant est une indication de la résistance de ladite souche *M. tuberculosis* au médicament antibactérien testé.

2. Procédé selon la revendication 1, dans lequel lesdits intervalles de temps fixes à l'étape C) sont de 1 à 5 minutes.

3. Procédé selon les revendications 1 ou 2, dans lequel les intervalles de temps fixes démarrent 2 minutes après l'addition respective.

4. Procédé selon la revendication 3, dans lequel lesdits intervalles de temps fixes de l'étape C) sont de 2 minutes.

5. Procédé selon la revendication 4, dans lequel ladite période de temps totale de l'étape C) est de 10 à 30 minutes, de préférence de 20 minutes.

6. Procédé selon les revendications 1 ou 5, dans lequel le temps d'incubation choisi à l'étape B) est de 8 à 36 heures, de préférence de 24 heures.

7. Procédé selon la revendication 6, dans lequel chacun desdits médicaments antibactériens de l'étape B) est choisi dans le groupe constitué de la rifampicine, de l'isoniazide, de l'éthambutol, du pyrazinamide, de l'ofloxacine, de la moxifloxacine, de l'amikacine, de la kanamicine, de la capréomycine, du linézolide, de la clofazimine et du méropénème.

8. Procédé selon la revendication 7, dans lequel lesdits médicaments antibactériens proposés sont la rifampicine, la moxifloxacine et l'amikacine.

9. Procédé selon les revendications 1 ou 8, dans lequel ladite sonde de spin est la 1-hydroxy-3-carboxy-2,2,5,5-tétraméthylpyrrolidine (CPH).

10. Procédé selon les revendications 1 ou 7, dans lequel lesdites autres sondes de spin utilisées pour la détection du ROS dans des techniques EPR sont choisies dans le groupe des hydroxylamines cycliques.
